Europäisches Patentamt

European Patent Office

Office européen des brevets

(19)

(11) Numéro de publication: **0 048 187**
**B1**

(12)

# FASCICULE DE BREVET EUROPEEN

(45) Date de publication du fascicule du brevet:
**02.04.86**

(51) Int. Cl.⁴: **A 61 B 5/00**

(21) Numéro de dépôt: **81401137.5**

(22) Date de dépôt: **17.07.81**

(54) Installation pour la surveillance cardiologique de patients.

(30) Priorité: **12.09.80 FR 8019691**
**28.04.81 FR 8108394**

(43) Date de publication de la demande:
**24.03.82 Bulletin 82/12**

(45) Mention de la délivrance du brevet:
**02.04.86 Bulletin 86/14**

(84) Etats contractants désignés:
**AT BE CH DE GB IT LI LU NL SE**

(56) Documents cités:
**US - A - 3 572 316**
**US - A - 3 768 017**
**US - A - 3 882 277**
**US - A - 3 902 478**
**US - A - 4 121 573**

(73) Titulaire: **SOCIETE D'ETUDES ET D'INFORMATIQUE POUR LA RECHERCHE MEDICALE ET INDUSTRIELLE S.E.I.R.M.I., 14 rue Pasteur, F-92210 Saint Cloud (Hauts de Seine) (GB)**

(72) Inventeur: **Mugica, Jacques Edmond, 72, Avenue Henri Martin, F-75016 Paris (FR)**
Inventeur: **Casali, Ettore Guido Attilo, 265, rue du Fg Saint Antoine, F-75011 Paris (FR)**

(74) Mandataire: **Rataboul, Michel, Cabinet Michel Rataboul 69, rue de Richelieu, F-75002 Paris (FR)**

# Description

On sait que les malades atteints de certaines affections cardiaques doivent être soumis à une surveillance constante à l'égard de certains paramètres cardiologiques.

Pour cela, on utilise des électrodes qui sont reliées par des conducteurs à un appareil transcodeur qui transforme les signaux reçus des électrodes par les conducteurs en des informations significatives et humainement compréhensibles tel qu'un graphique inscrit sur un oscillographe cathodique.

Les conducteurs sont plus ou moins longs selon la distance qui sépare le patient de l'appareil transcodeur mais, dans la pratique, il est impossible de placer cet appareil dans un local différent de celui dans lequel se trouve le patient, à savoir la plupart du temps, une chambre d'un établissement hospitalier.

Une telle installation présente de nombreux inconvénients car les informations provenant des électrodes sont directement appliquées à l'appareil transcodeur de sorte que les mouvements du patient ou les déplacements accidentels des conducteurs provoquent fréquemment des tractions mécaniques provoquant parfois des fausses alarmes, des déréglages ou des parasites.

De plus, on comprend aisément que le patient est pour ainsi dire immobilisé puisque même si son état de santé le lui permet, il lui est impossible de quitter le local dans lequel se trouve l'appareil, par exemple pour faire une promenade dans le jardin ou dans une autre pièce de l'établissement hospitalier.

Enfin, la présence obligatoire d'un appareil transcodeur dans chaque chambre de malade impose au personnel hospitalier des rondes permanentes pour aller vérifier sur place les signaux transcrits par l'appareil.

Pour remédier à ces inconvénients on a déjà pensé à utiliser des signaux radio mais ces installations ne donnent pas entière satisfaction car elles supposent une infrastructure spécialement conçue.

Ainsi le brevet US 3 572 316 décrit une installation qui comprend d'une part un ensemble porté par le patient et composé de capteurs et d'un émetteur et, d'autre part, un récepteur placé dans la même pièce que celle où se trouve le patient. Ce récepteur envoie dans un réseau fixe (de distribution d'électricité, par exemple) un courant spécialement modulé. Puis, sur ce même réseau est branché un transcodeur placé dans une autre pièce. Il y a donc transformation des informations radio en informations électriques d'un type spécialement conçu par référence à d'autres courants coexistants sur le même réseau. Cette installation suppose un câble souple entre le récepteur et le réseau, les fils du réseau et un câble souple entre le réseau et le transcodeur.

Le récepteur et le transcodeur doivent obligatoirement être conçus l'un pour l'autre et le transcodeur ne peut pas fonctionner dans d'autres conditions.

On connaît également le brevet US 4 121 573 qui décrit une installation selon le préambule de la revendication 1 comprenant un émetteur radio et un récepteur relié par un câble souple à un transcodeur pré-existant.

Mais il s'agit d'un montage où on fait usage d'une simple prise («plug» 60) pour raccorder le récepteur à différents appareils successifs (colonne 4 lignes 29 à 31) qui doivent, chacun, comprendre une prise femelle supplémentaire et donc distincte de l'alimentation standard par trois câbles. On ne peut donc pas à l'inverse brancher le cordon 14 sur l'émetteur 10 pour obtenir un fonctionnement normal.

Avec l'invention, telle qu'elle est revendiquée dans les revendications, on peut au contraire relier directement deux des trois prises mâles 12, 13 et 14 aux électrodes 1 et 2, et la troisième prise mâle à une autre électrode pour retrouver le fonctionnement d'origine. Cela permet, par exemple, un dépannage immédiat en cas de panne radio.

On connaît également d'autres brevets qui prévoient l'émission par radio d'informations correspondant à des paramètres physiologiques.

La plupart d'entre eux prévoient deux électrodes seulement. Dans ce cas l'une d'elles est reliée au pôle négatif d'une source de courant continu et l'autre est donc seule à être réellement fonctionnelle c'est-à-dire connectée à un amplificateur et à l'émetteur.

D'autres systèmes connus pour remédier à ce défaut prévoient que deux électrodes sont connectées à un amplificateur différentiel pour tenir compte de la différence des valeurs de signaux sur les deux électrodes, quelles que soient ces valeurs absolues. Mais, alors, il faut une troisième électrode pour constituer une base de mesure. Cela constitue une gêne supplémentaire pour le patient et une source d'incident de plus puisqu'il y a trois électrodes et trois fils au lieu de deux.

Par ailleurs, l'usage obligatoire d'une antenne pour toute transmission radio se traduit dans la pratique par la présence d'un autre fil supplémentaire qui sort de l'émetteur et pend librement mais crée une gêne incontestable à l'utilisateur à moins de se contenter d'une antenne intégrée de faible portée.

Lorsqu'un établissement hospitalier est équipé avec une telle installation, il ne peut la modifier ou la moderniser que s'il change à la fois tous les émetteurs et tous les appareils récepteurs-transcodeurs, c'est-à-dire la totalité des appareils constituant l'installation.

La présente invention concerne une installation du type à émetteur de signaux radio considérablement plus économique et rationnelle grâce à une combinaison judicieuse des installations à conducteurs électriques (électrodes et transcodeurs) et des installations à émission radio.

L'invention sera bien comprise par la description détaillée ci-après faite en référence au dessin annexé. Bien entendu, la description et le dessin ne sont donnés qu'à titre d'exemple indicatif et non limitatif.

La fig. 1 est une vue schématique d'une installation conforme à l'invention.

La fig. 2 est une vue schématique montrant les possibilités d'utilisation de plusieurs installations conformes à l'invention groupées dans un seul et même local.

La fig. 3 représente un schéma partiel des circuits de l'émetteur 5 qui illustre un mode particulier de réalisation de l'invention selon laquelle l'installation ne comprend que deux électrodes et deux fils.

La fig. 4 est un schéma partiel des circuits de l'émetteur montrant un mode de réalisation de l'antenne émettrice.

La fig. 5 est un schéma d'un autre mode de réalisation de l'antenne émettrice.

La fig. 6 est un schéma partiel de l'émetteur montrant le détail de réalisation d'un dispositif d'arrêt et de mise en route automatique dont l'émetteur est muni.

En se reportant au dessin, on voit qu'une installation conforme à l'invention est destinée à la surveillance cardiologique de patients et est du type comprenant d'une part un émetteur de signaux radio 5 qui est relié par des fils 3 et 4 à des électrodes 1 et 2 et qui doit être porté par un patient et, d'autre part, un récepteur de ces signaux radio et un transcodeur 8 de signaux électriques en informations significatives et humainement compréhensibles. Le transcodeur 8 est du type standard et comprend trois conducteurs souples 15, 16 et 17 munis d'extrémités mâles 12, 13 et 14 destinées à être raccordées à des extrémités femelles de conducteurs souples reliés aux électrodes 1 et 2. Le récepteur 7 est autonome par rapport au transcodeur 8 et comprend trois prises femelles 9, 10 et 11 qui peuvent être raccordées aux extrémités mâles 12, 13 et 14, l'une d'elles étant à la masse et qui sont associées à des moyens (connus en soi) prévus dans le récepteur 7 pour reconstituer à sa sortie en partant des signaux radio reçus de l'émetteur 5 les signaux électriques existant à la sortie des câbles d'électrodes.

Il suffit de deux électrodes cutanées 1 et 2 placées sur la poitrine du patient de sorte que l'émetteur 5 est simple, compact et très léger et peut être aisément porté par le patient, par exemple dans la poche d'un vêtement tel qu'un pyjama.

De ce fait, les fils 3 et 4 qui relient respectivement l'électrode 1 et l'électrode 2 à l'émetteur 5 sont très courts et ont, de toutes façons, moins d'un mètre. Des essais concluants ont été effectués avec des fils qui ont environ quarante centimètres.

Le patient ainsi équipé est tout à fait indépendant et les fils 3 et 4 sont suffisamment légers pour être convenablement placés afin de n'effectuer aucune traction sur les électrodes 1 et 2 fixées sans défaut sur la poitrine du patient.

Par ailleurs, dans un local qui est de préférence différent de celui dans lequel le patient est normalement situé, se trouve un récepteur autonome 7 accordé sur l'émetteur 5 et muni de trois prises femelles 9, 10 et 11. Le récepteur 7 est un appareil dont les composants électroniques sont bien connus de l'homme de métier et n'ont pas besoin, de ce fait, d'être décrits en détail. Les signaux radio reçus par le récepteur 7 des électrodes 1 et 2 via l'émetteur 5, sont transformés en signaux électriques et conduits aux prises femelles 9, 10 et 11.

Le récepteur 7 est un appareil léger et compact qui peut par exemple, être placé directement sur l'appareil transcodeur 8 (généralement nommé «moniteur») quel que soit le type de ce dernier, dès lors que l'entrée des informations dans ce dernier se fait par trois conducteurs électriques 15, 16 et 17 munis chacun de prises mâles normalisées respectivement 12, 13 et 14.

Il suffit, alors de placer les prises mâles 12, 13 et 14 dans les prises femelles 9, 10 et 11 du récepteur 7 pour que l'appareil transcodeur 8 reçoive les informations voulues et les transforme, comme cela est connu en soit, en informations significatives et humainement compréhensibles et, cela, sans aucune adaptation et sans aucun réglage spécifique.

Les prises femelles 9, 10 et 11 sont exactement équivalentes à celles des conducteurs supprimés et qui aboutissaient, avant transformation directement aux électrodes.

L'ensemble de captation et d'émission (électrodes 1 et 2, fils 3 et 4 et émetteur 5) est trè simple et léger de sorte qu'il libère le malade de toute espèce d'entraves ou de gênes dans son comportement physique.

Quant à l'ensemble de réception, de transcodage et de surveillance, il est scindé en deux parties indépendantes et facilement connectables et déconnectables puisque le récepteur 7 est autonome et peut être associé simplement par les prises femelles 9, 10 et 11 d'une part et mâles 12, 13, et 14 d'autre part à un appareil transcodeur 8 de n'importe quel type connu du fait de la normalisation de ses prises.

Grâce à la constitution de cet ensemble réception-transcodage, il est possible à un établissement hospitalier de s'équiper d'émetteurs 5 et de récepteurs 7 sans devoir éliminer les appareils transcodeurs dont il dispose.

La légèreté et la stabilité de l'ensemble de captation et d'émission ont pour avantage de supprimer les tractions mécaniques sur les fils, ce qui évite les déconnections des électrodes ainsi que l'émission de signaux signifiant des anomalies dans la morphologie du signal électro-cardiographique alors qu'en réalité le patient se trouve dans une situation normale.

La simplification de l'émetteur 5 permet, notamment, d'utiliser une source d'énergie légère telle qu'une pile classique de neuf Volts.

La stabilité de cet ensemble élimine la création de parasites, de sorte que le signal qui constitue l'information perçue par le personnel constitue l'image exacte et détaillée de la morphologie électro-cardiographique du patient, ce qui accroît la qualité de la surveillance et donne plus d'assurance au personnel hospitalier.

Grâce aux qualités techniques des informations disponibles, l'installation conforme à l'invention peut être associée à des ensembles électroniques de grande qualité tels que des ordinateurs car les opérations effectuées par ceux-ci deviennent plus fiables puisqu'elles sont effectuées à partir d'éléments purs (sans parasites).

On peut, alors, informatiser les paramètres électro-cardiographiques même particulièrement complexes ou anormaux y compris lorsque le patient est muni d'un stimulateur cardiaque dont l'impulsion peut elle-même être surveillée et prise en compte sans qu'aucune confusion puisse se produire et sans création de signaux parasites, quel que soit le voltage du stimulateur.

Plusieurs couples récepteur-transcodeur peuvent être placés dans un même local, chacun de ces couples recevant un moyen d'identification de l'émetteur correspondant et, par suite, du patient ainsi surveillé à distance.

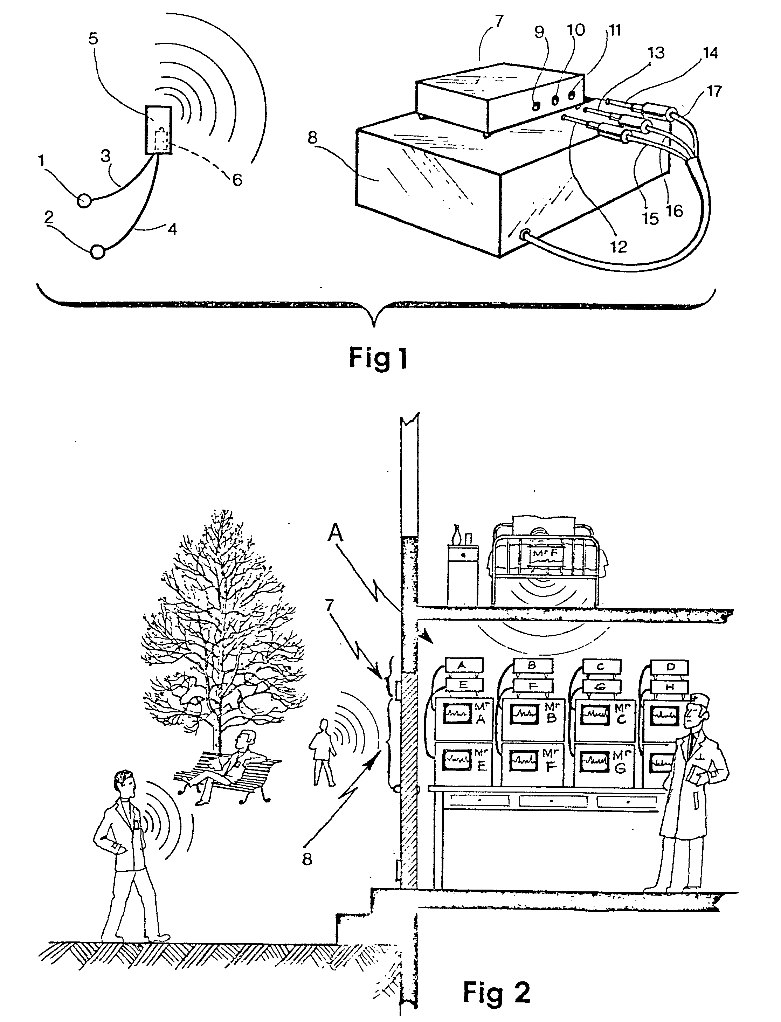

La fig. 2 illustre une telle installation et l'on voit que lorsque les circonstances le permettent, les patients dont l'état de santé est tel qu'ils peuvent sortir de leur chambre aussi bien que ceux qui sont obligés de rester alités, conservent chacun leur autonomie et peuvent, par exemple, se trouver dans un jardin ou dans un local autre que leur chambre (bibliothèque, salle de télévision, salle de visite, etc...).

En effet, dans le local de surveillance A se trouvent plusieurs couples récepteur 7 – appareils transcodeurs 8 reliés par couple au moyen de cordons électriques contenant chacun les trois conducteurs 15, 16 et 17 déjà décrits et représentés sur la fig. 1.

Chaque couple, et de préférence, chaque appareil transcodeur 8, reçoit un moyen d'identification tel que le nom du malade ou le numéro qui lui est attribué afin que le personnel de surveillance puisse immédiatement identifier le patient dont l'émetteur 5 provoque une information alarmante ou anormale sur un des appareils transcodeurs 8.

Naturellement, dans la pratique, il peut s'avérer indispensable d'imposer au malade un minimum de discipline et, par exemple, d'informer le personnel hospitalier avant de quitter la chambre ou de sortir dans le jardin afin que le personnel sache rapidement où se transporter si nécessaire, c'est-à-dire si un signal grave se produit.

De toutes façons, étant donné les caractéristiques de la pathologie en cause, il est clair que les patients ne doivent jamais être très éloignés de l'établissement hospitalier et même des moyens de traitement, de sorte que l'émetteur 5 peut être de très faible puissance pour n'avoir une portée que de quelques dizaines de mètres.

L'émetteur peut être associé à un système de recherche et repérage par lequel le personnel de surveillance est informé de l'endroit au moins approximatif où se trouve le porteur de l'émetteur 5.

De même, au lieu de réaliser les récepteurs 7 sous forme d'un ensemble électronique placé dans un boîtier, on peut prévoir un coffret comprenant plusieurs tiroirs ou «racks» non carossés et correspondant chacun aux circuits de réception.

Afin de capter, transmettre et obtenir des signaux précis et dans leur intégralité, et en particulier les signaux provoqués par un stimulateur cardiaque, on utilise selon l'invention des circuits électroniques tels qu'il admettent des signaux susceptibles de se situer dans une plage de fréquences appelée «bande passante» de 0,05 à 4000 périodes sans atténuation.

Les électrodes 1 et 2 placées sur le corps du patient captent des manifestations physiologiques du patient telles que l'activité cardiaque (spontanée ou provoquée par un stimulateur). La respiration, la température, les pressions de toutes sortes, etc... peuvent également être captées dès lors que l'on utilise des capteurs appropriés.

Ici, l'exemple retenu est celui de la captation des signaux résultant de l'activité cardiaque.

Il résulte de la captation par les électrodes 1 et 2 un courant électrique modulé dans les fils 3 et 4 qui constitue les signaux de base devant être observés et analysés.

Pour cela, ces signaux doivent être amplifiés.

Comme on l'a dit plus haut, les dispositifs de bonne qualité connus jusqu'à ce jour comprennent obligatoirement trois fils et trois électrodes, la troisième consistant en une mise à la masse pour fermer le circuit et constituer une sorte de référence, ou base, au courant qui circule dans les fils 3 et 4.

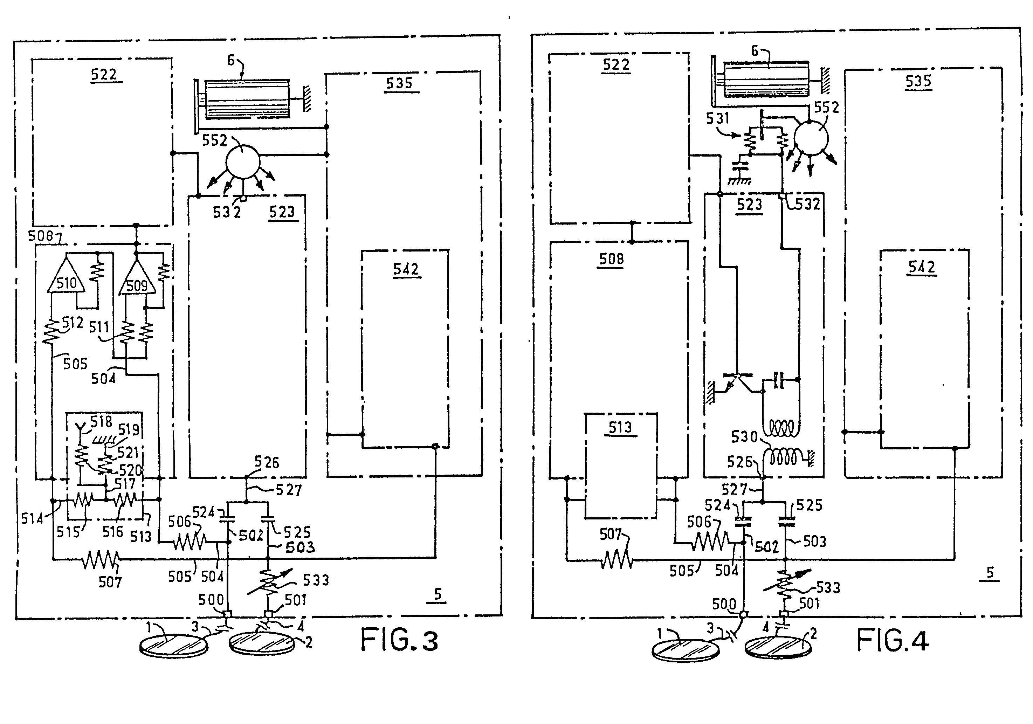

Sur la fig. 3, on voit que les fils 3 et 4 aboutissent à des bornes 500 et 501 du boîtier de l'appareil désigné par la référence générale 5.

A ces bornes 500 et 501 correspondent des conducteurs 502 et 503 d'où partent des lignes 504 et 505 comprenant des résistances de protection respectivement 506 et 507 et qui aboutissent à un ensemble amplificateur 508.

Ici, l'ensemble amplificateur 508 comprend deux amplificateurs 509 et 510 montés en différentiel.

Les lignes 504 et 505 aboutissent aux pôles positifs respectivement de l'amplificateur 509 et de l'amplificateur 510 avec interposition de résistance de valeur importante respectivement 511 et 512.

Les deux liaisons 504 et 505 sont associées à un circuit 513 qui comprend une ligne 514 sur laquelle sont montées en série deux résistances 515 et 516.

Entre ces deux résistances 515 et 516, aboutit une liaison 517 d'un circuit qui comprend deux branches respectivement positive 518 et négative (à la masse) 519 sur chacune desquelles est intercalée une résistance respectivement 520 et 521.

On voit qu'ainsi le circuit 513 a pour effet de créer une tension qui est appliquée entre les résistances 515 et 516 sur la ligne de jonction 514 entre les deux liaisons 504 et 505.

De ce fait, on assure entre les résistances 515 et 516 une tension nulle servant de référence aux amplificateurs 509 et 510, ce qui supprime la nécessité d'un troisième fil et d'une troisième électrode.

Les amplificateurs 509 et 510 envoient un signal dans un modulateur 522 de tout type connu associé à un oscillateur 523 constituant l'émetteur proprement dit.

L'antenne émettrice est constituée par la jonction des électrodes 1 et 2 à l'émetteur 5.

Une première variante de réalisation de ce montage est représentée sur la fig. 4 où l'on voit que des condensateurs 524 et 525 sont intercalés sur les conducteurs 502 et 503, au-delà de leur jonction avec les liaisons 504 et 505 et que la sortie 526 et l'oscillateur 523 comporte une liaison 527 avec lesdits conducteurs 502 et 503 au-delà des condensateurs 524 et 525.

Avec ce montage, les signaux électriques arrivant des électrodes 1 et 2 par les fils 3 et 4 empruntent les liaisons 504 et 505 puisque les condensateurs 524 et 525 interrompent la continuité électrique des conducteurs 502 et 503.

Les signaux en haute fréquence issus de l'oscillateur 523 sont envoyés dans les conducteurs 502 et 503 par la liaison 527 et atteignent les fils 3 et 4 puisque les condensateurs 524 et 525 ne constituent pas un obstacle au parcours de ces signaux.

Les résistances 506 et 507, en revanche, empêchent la propagation de ces signaux et protègent ainsi l'ensemble amplificateur 508.

On note que la présence des condensateurs 524 et 525 a pour effet de mettre au même potentiel les deux fils 3 et 4 à l'égard des signaux haute fréquence.

Grâce à ce montage, les signaux sont émis par les fils 3 et 4 eux-mêmes qui jouent le rôle d'antenne, de sorte qu'il n'est pas nécessaire de prévoir une antenne supplémentaire qui constituerait une gêne pour le porteur de l'émetteur.

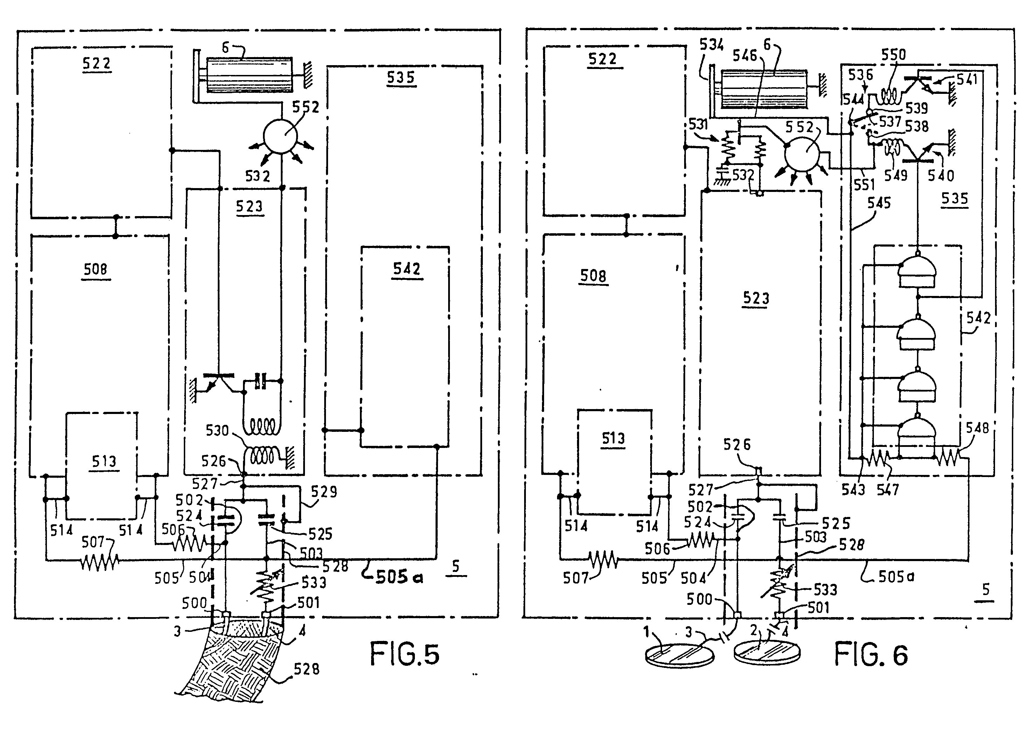

Sur la fig. 5, on a représenté une autre variante selon laquelle les fils 3 et 4 sont situés, de manière connue en soi, dans une tresse de blindage 528 qui se prolonge autour des conducteurs 502 et 503.

La liaison 527 est reliée à la tresse 528 par une dérivation 529 de sorte que, finalement, l'antenne est constituée à la fois par les fils 3 et 4 et par la tresse 528.

Toutefois, on pourrait se contenter de constituer l'antenne par les fils 3 et 4 seulement.

Mais l'expérience montre qu'il peut être intéressant d'utiliser le montage représenté sur la fig. 5 car la portée de l'antenne est ainsi plus grande que si l'on n'utilise que les fils 3 et 4 seuls.

On note que la tresse blindée 528 a pour effet de mettre les fils 3 et 4 à l'abri des parasites émis principalement par le courant électrique à 50 périodes présent dans les installations électriques de tous les locaux.

Avec ce montage, on élimine ces parasites et l'on constitue une antenne à la fois simple et très efficace puisqu'il n'y a qu'un seul cordon qui s'étend depuis l'émetteur 5 proprement dit jusqu'à une très courte distance en deçà des électrodes 1 et 2 qui, elles, sont situées sur les fils 3 et 4 rendus indépendants à leur voisinage immédiat.

En reliant la sortie 526 de l'oscillateur 523 à la tresse de blindage 528, cette dernière est portée au potentiel haute fréquence alors que par rapport au courant alternatif 50 périodes elle est à la masse, étant donné que le secondaire 530 du transformateur haute fréquence a une impédance négligeable et qu'il est relié à la masse.

Comme l'émetteur 5 doit être très léger et aussi peu volumineux que possible, le générateur d'énergie 6, qui est une pile ou un accumulateur, doit lui-même être aussi peu volumineux que possible.

Il est donc très important d'utiliser son énergie d'une manière aussi performante que possible car si la puissance de ce générateur 6 venait à baisser très rapidement, les signaux émis pourraient être soit perturbés, soit rendus si faibles qu'ils seraient mal perçus par le récepteur.

On parvient à la fois à utiliser un générateur de faible volume et à garantir une utilisation constante jusqu'à un seuil inférieur qui, lorsqu'il est atteint, signifie que la pile doit être changée, tandis qu'avant d'atteindre ce seuil le fonctionnement est invariable.

Sur la fig. 4, on voit un tel montage selon lequel on intercale un semi-conducteur du type à effet de champ 531 entre le générateur 6 et l'entrée 532 de l'oscillateur 523.

Comme on le sait, un semi-conducteur à effet de champ a pour caractéristique de présenter une résistance qui est inversement proportionnelle au courant d'entrée qu'on lui applique.

Il en résulte que l'oscillateur 523 reçoit à son entrée 532 une alimentation constante alors que la tension du courant débité par le générateur 6 diminue régulièrement.

Si on utilise une pile de 9 Volts, par exemple, on sait que sa tension diminue régulièrement avec le temps d'usage et l'on sait que l'ensemble des organes soumis à cette alimentation présentera des anomalies de fonctionnement lorsqu'un seuil minimum sera atteint.

On élimine totalement cet inconvénient avec le montage qui vient d'être décrit puisque le semi-conducteur à effet de champ a pour effet de maintenir la tension nominale (9 Volts dans le présent exemple) à l'entrée 532 de l'oscillateur 523 lorsque la tension de la pile 6 baisse jusqu'à un certain seuil prédéterminé et situé immédiatement en amont de la valeur pour laquelle l'oscillateur ne fonctionne plus de manière sûre, auquel cas l'appareil est purement et simplement «arrêté» ce qui signifie qu'il faut changer la pile pour en mettre une neuve.

On note que ce montage est particulièrement intéressant car la stabilisation de la tension d'alimentation à la valeur nominale ne nécessite aucune consommation d'énergie extérieure puisque le semi-conducteur à effet de champ crée lui-même la variation de résistance qui est à l'origine même de la régulation.

Compte tenu qu'un émetteur conforme à un mode particulier de réalisation de l'invention est destiné à être porté par un patient qui est en mesure de se déplacer, l'émetteur est particulièrement soumis aux effets parasites du courant alternatif à 50 périodes utilisé dans les bâtiments.

Afin d'éliminer ces perturbations sur l'appareil et notamment sur les amplificateurs 509 et 510 montés en différentiel, on prévoit une résistance 533 montée en série sur le conducteur 503, immédiatement après la borne 501, cette résistance 533 étant du type réglable ce qui permet, par action sur ses organes de réglage, d'équilibrer les deux entrées de façon à faire fonctionner les amplificateurs 509 et 510 en «différentiel vrai» et à équilibrer d'éventuelles différences d'ondulations des signaux électriques sur l'un des deux fils 3–4.

Avant la mise en service de l'appareil, on relie les entrées (électrodes 1–2, fils 3–4, bornes 500–501) à une source de courant alternatif à 50 périodes et l'on agit sur les organes de réglage de la résistance 533 pour obtenir une différence nulle entre les deux amplificateurs 509 et 510.

Dès lors, en cours de fonctionnement, l'appareil sera insensible aux parasites de 50 périodes.

Naturellement, si l'appareil est soumis à un courant d'une autre fréquence que les 50 périodes indiquées plus haut, c'est à la fréquence de ce courant qu'il faut effectuer le réglage préalable (notamment 60 périodes).

Les conditions d'emploi de l'émetteur rappelées ci-dessus impliquent, comme on l'a dit, une utilisation optimale du générateur 6 et il est prévu conformément à un mode particulier de réalisation de l'invention, que l'émetteur 5 comprend un dispositif automatique pour que le générateur soit isolé lorsque les électrodes 1 et 2 ne sont plus en place tandis que le générateur 6 est remis en action automatiquement dès que les électrodes sont à nouveau remises en place.

On est ainsi assuré que le générateur 6 ne débite du courant que lorsque cela est nécessaire et sans que le personnel ait à agir sur un bouton traditionnel Marche-Arrêt.

En se reportant à la fig. 6, on voit comment on peut réaliser un tel dispositif automatique d'arrêt et de mise en marche.

La sortie de l'alimentation 534 du générateur 6 est reliée à un circuit 535 comprenant un relai bi-stable c'est-à-dire un interrupteur à deux positions stables 536 dont un contact mobile 537 est susceptible de coopérer soit avec un plot 538, soit avec un plot 539 selon la position dans laquelle il est sollicité à partir de semi-conducteurs 540 et 541 dont le débit est soumis à la polarisation de leur base par une logique 542.

L'entrée d'alimentation 543 de la logique 542 et le plot commun 544 de l'interrupteur 536 sont reliés par une ligne 545 à laquelle aboutit une ligne 546 provenant de la sortie d'alimentation 534 du générateur 6.

De la sorte, les différents éléments de la logique 542 sont tous alimentés en permanence et directement par le générateur 6 et se trouvent ainsi polarisés en +.

Par ailleurs, une ligne 505a relie le conducteur 503 et une résistance 547 dont l'une des extrémités est reliée à l'entrée d'alimentation 543 et qui est située au-delà d'une résistance 548, ces deux résistances 547 et 548 étant montées en série.

Les éléments de la logique 542, constitués par des bascules, sont par ailleurs montés en série et connectés à la liaison 505a entre les deux résistances 547 et 548.

Le montage des deux transistors 540 et 541, tel qu'il est représenté sur la fig. 4, montre que le collecteur de chacun d'eux est relié à une bobine de self 549 et 550 tandis que leur émetteur est à la masse. Leur base est connectée à la logique 542 à des emplacements correspondants à des polarités contraires dans une position donnée des bascules.

Entre la self 549 et le plot 538, est connectée une liaison 551 qui aboutit à l'alimentation générale 552 de l'émetteur 5, et notamment de l'oscillateur 523, (semi-conducteur à effet de champ 531).

Le fonctionnement de ce montage est le suivant: lorsque les électrodes 1 et 2 sont débranchées, l'entrée d'alimentation 543 de la logique 542 est polarisée en + puisqu'elle est reliée directement à la sortie d'alimentation 534 du générateur 6.

La position des bascules est telle que le transistor 540 débite et oblige le contact mobile 537 à s'appliquer contre le plot 539 dans la position représentée en trait plein sur la fig. 6.

L'ouverture de l'interrupteur 536 a ainsi pour conséquence d'interrompre le passage du courant depuis la source 534 jusqu'à l'alimentation générale 552 puisque les lignes 546 et 551 n'ont plus de continuité.

Dans cette situation, le générateur 6 est complètement isolé par rapport aux différents organes de l'émetteur, du fait que le basculement du contact mobile 537 a rompu la continuité électrique des lignes 546 et 551 entre le générateur 6 et l'alimentation générale 552.

Le transistor 540 n'est plus alimenté du côté de son collecteur et la seule consommation permanente est alors celle de la base du transistor 540, consommation qui est tout à fait négligeable.

Le contact mobile 537 assure le passage du courant par la ligne 546 et par les plots 544 et 539, de sorte que le transistor 541 est alimenté du côté de son collecteur mais sa base étant au potentiel zéro, il ne débite pas.

La position représentée sur la fig. 6 est donc stable et la consommation de l'appareil négligeable.

Lorsque les électrodes 1 et 2 sont mises en place, leur résistance propre est faible par rapport à la valeur de la résistance 547 puisqu'elles sont au contact du patient qui est conducteur.

En effet, la résistance 547 a une valeur importante, de sorte que la tension d'entrée dans la logique 542 par les connections situées entre les résistances 547 et 548 tombe à zéro, ce qui provoque le changement de sens des bascules de la logique 542.

Les polarités changent et le transistor 541 débite, de sorte que le contact mobile 537 quitte sa position représentée en trait plein sur la fig. 4 pour prendre sa position représentée en pointillé contre le plot 538.

L'interruption qui en résulte entre les plots 544 et 539 a pour conséquence que le transistor 541 n'est plus alimenté et, de ce fait, ne débite plus.

C'est au contraire le transistor 540 qui est maintenant alimenté et, simultanément, la continuité des lignes 546 et 551 est reconstituée de sorte que l'alimentation générale 552 est à nouveau rétablie.

Le transistor 540 est prêt à déplacer à nouveau le contact mobile 537 dès que les électrodes 1 et 2 seront à nouveau séparées.

Naturellement, on pourrait utiliser un montage différent pour réaliser ce dispositif de mise en action et d'arrêt automatique. Celui qui a été représenté et décrit ici présente l'avantage de ne nécessiter qu'une consommation d'énergie insignifiante pendant qu'il est en «état de veille» c'est-à-dire pendant que les électrodes sont séparées et que l'appareil n'est pas utilisé. En outre, il ne perturbe pas le fonctionnement normal de l'émetteur 5.

L'intérêt d'un tel dispositif est évident puisqu'il élimine toute possibilité d'erreur ou d'omission de la part de la personne qui place et retire l'émetteur, contrairement à ce qui se passe actuellement où il est fréquent d'oublier d'agir sur le bouton d'arrêt, ou de retirer la pile, de sorte que le générateur fournit de l'énergie en pure perte.

Il ressort de la description ci-dessus que l'invention permet d'utiliser les trois câbles standards d'un transcodeur, sans aucune adaptation, en reconstituant à l'entrée de ces câbles les signaux existant à la sortie des câbles d'électrodes.

Le transcodeur ne peut donc être qu'un «moniteur» ayant trois câbles d'entrée. L'émetteur doit avoir des électrodes situées à l'extrémité de fils adaptés à constituer une antenne.

Avec ce montage on assure la continuité radio depuis les électrodes jusqu'à l'entrée des câbles du moniteur, alors que celui-ci ne peut à l'origine, fonctionner que par continuité électrique le long de câbles («cordon sujet»).

L'invention n'est pas limitée au seul mode de réalisation décrit et représenté mais en embrasse, au contraire, toutes les variantes.

On peut souligner, notamment, que le récepteur 7 peut être associé au transcodeur 8 («moniteur») non seulement en le posant à côté de lui mais également par des moyens d'assujetissement: glissières, encastrement, tiroirs, etc...

**Revendications**

1. Installation pour la surveillance cardiologique de patients, du type comprenant d'une part un émetteur de signaux radio (5) qui est relié par des fils (3 et 4) à des électrodes (1 et 2) et qui doit être porté par un patient et, d'autre part, un récepteur (7) transformant ces signaux radio en signaux électriques et un transcodeur (8) transformant ces signaux électriques en informations significatives et humainement compréhensibles, caractérisée en ce que le transcodeur (8) est du type standard, qui peut être raccordé directement aux électrodes, que l'entrée du transcodeur est constituée par trois conducteurs souples (15, 16 et 17) munis d'extrémités mâles (12, 13 et 14) destinées à être raccordées à des extrémités femelles de câbles souples reliés aux électrodes (1 et 2), ou bien à trois prises femelles (9, 10 et 11) constituant la sortie du récepteur et que le récepteur (7) est autonome par rapport au transcodeur et comprend des moyens pour reconstituer à la sortie du récepteur en partant des signaux radio reçus de l'émetteur (5) les signaux électriques existant à la sortie des câbles d'électrodes, en permettant ainsi un dépannage immédiat en cas de panne de l'émetteur et/ou du récepteur.

2. Installation selon la revendication 1, caractérisée en ce que les circuits de l'émetteur (5) de signaux radio comprennent un ensemble amplificateur (508) constitué de deux amplificateurs (509 et 510) connectés de manière différentielle et dont le pôle positif est relié par des liaisons (504 et 505) à des bornes (500 et 501) des fils (3 et 4) avec interposition de résistances (511 et 512) de valeur importante, lesdites deux liaisons (504 et 505) étant associées à un circuit (513) qui comprend une ligne (514) comprenant deux résistances (515 et 516) entre lesquelles aboutit une liaison (517) d'un circuit contenant une résistance (520–521) sur chacune de ses deux branches positive (518) et négative (519).

3. Installation selon la revendication 1, caractérisée en ce que les circuits de l'émetteur (5) de signaux radio comprenant un ensemble amplificateur (508) auquel aboutissent, par des liaisons (504 et 505), des conducteurs (502 et 503) prolongeant les fils (3 et 4), une résistance réglable (533) est montée en série sur l'un des éléments conducteurs (502, 503, 504 ou 505) situé entre une borne (500 ou 501) et l'ensemble amplificateur (508).

4. Installation selon la revendication 1, caractérisée en ce que l'émetteur (5) comprend un dispositif automatique selon lequel le générateur d'énergie (6) est soit isolé quand les électrodes (1 et 2) ne sont plus en place sur le corps du patient, soit en circuit quand les électrodes (1 et 2) sont en place.

5. Installation selon la revendication 4, caractérisée en ce que la sortie d'alimentation (534) du générateur (6) est reliée à un circuit (535) comprenant un interrupteur (536) à deux positions stables dont un contact mobile (537) est sollicité vers ses positions stables par des semi-conducteurs (540 et 541) dont le débit est soumis à la polarisation de leur base par une logique (542) dont l'entrée d'alimentation (543) est reliée directement en permanence à la sortie (534) du générateur (6) et qui est connectée à une liaison (505a) avec l'un des conducteurs (503) correspondant à l'une des électrodes (2).

**Claims**

1. System for cardiac monitoring of patients, of the kind comprising on the one hand a radio signal transmitter (5) which is coupled by wires (3 and 4) to electrodes (1 and 2) and which has to be worn by a patient and, on the other hand, a receiver (7) converting these radio signals and a transcoder (8) converting these electrical signals into electrical signals into significant and humanly understandable data, characterised in that the transcoder (8) is of the standard kind which may be coupled directly to the electrodes, that the unput of the transcoder is formed by three flexible conductors (15, 16 and 17) provided with plug-in extremities (12, 13 and 14) intended to be coupled to socket extremities of flexible leads connected to the electrodes (1 and 2) or else to three plug sockets (9, 10 and 11) forming the output of the receiver, and that the receiver (7) is independent with respect to the transcoder and comprises means for reconstituting, at the output of the receiver, from the radio signals received from the transmitter (5), the electrical signals present at the output of the electrode leads thus allowing an immediate standby operation in case of failure of the transmitter and/or of the receiver.

2. System according to claim 1, characterised in that the circuits of the radio signal transmitter (5) comprise an amplifier assembly (508) formed by two amplifiers (509 and 510) connected in a differential manner and of which the positive terminal is coupled by connections (504 and 505) to terminals (500 and 501) of the wires (3 and 4) with interposition of high rating resistors (511 and 512), the said two connections (504 and 505) being associated with a circuit (513) which comprises a channel (514) comprising two resistors (515 and 516) between which terminates a connection (517) from a circuit containing a resistor (520–521) in each of its positive (518) and negative branches (519).

3. System according to claim 1, characterised in that the circuits of the radio signal transmitter (5) comprise an amplifier assembly (508) reached, via connections (504 and 505), by conductors (502 and 503) extending the wires (3 and 4), a variable resistor (533) being installed in series with one of the conductive elements (502, 503, 504 and 505) situated between a terminal (500 or 501) and the amplifier assembly (508).

4. System according to claim 1, characterised in that the transmitter (5) comprises an automatic device whereby the energy generator (6) is either cut off when the electrodes (1 and 2) are no longer in position on the patient's body, or in circuit when the electrodes (1 and 2) are in position.

5. System according to claim 4, characterised in that the supply output (534) of the generator (6) is connected to a circuit (535) comprising a switch (536) having two stable positions, of which a movable contact (537) is urged towards its stable positions by semiconductors (540 and 541) of which the output is subjected to the polarisation of their base via logic (542) of which the supply input (543) is permanently connected directly to the output (534) of the generator (6) and which is coupled with a connection (505a) to one of the conductors (503) corresponding to one of the electrodes (2).

**Patentansprüche**

1. Gerät zur Herzüberwachung von Patienten, mit einem Funksignalsender (5), der über Kabel (3) und (4) an Elektroden (1 and 2) angeschlossen ist und der vom Patienten getragen wird, einerseits und mit einem Empfänger (7) anderseits, der diese Funksignale in elektrische Signale umsetzt und einem Transkodierer (8), der diese elektrischen Signale in bezeichnende und vom Menschen erfassbare Informationen umsetzt, dadurch gekennzeichnet, dass der Transkodierer (8) ein Standardtranskodierer ist, der direkt an die Elektroden angeschlossen werden kann, dass der Eingang des Transkodierers aus drei biegsamen Leitern (15, 16 und 17) besteht, die an ihren Enden mit Steckern (12, 13 und 14) versehen sind, die mit Muffen biegsamer Kabel verbunden sind, die an die Elektroden (1 und 2) angeschlossen sind, ober die mit drei Muffen (9, 10 und 11) verbunden sind, die den Ausgang des Empfängers bilden, und dadurch, dass der Empfänger (7) unabhängig vom Transkodierer ist und Mittel aufweist, um am Ausgang des Empfängers, ausgehend von vom Sender (5) empfangenen Funksignalen elektrische Signale zu rekonstituieren, die an den Enden der Elektrodenkabel anliegen, wodurch bei einer Panne des Senders und/oder des Empfängers eine sofortige Störungsbeseitigung emöglicht wird.

2. Gerät nach Anspruch 1, dadurch gekennzeichnet, dass die Schaltung des Senders (5) der Funksignale eine Verstärkeranordnung (508) aufweist, die aus zwei Verstärkern (509 und 510) besteht, die in Differenzschaltung zueinander geschaltet sind und deren positiver Pol über Verbindungen (504 und 505) an Klemmen (500 und 501) von Kabeln (3 und 4) angeschlossen ist, wobei hochohmige Widerstände (511 und 512) an die Kabel geschaltet sind und die beiden Verbindungen (504 und 505) an eine Schaltung (513) angeschlossen sind, die eine Leitung (514) mit zwei Widerständen (515 und 516) aufweist, zwischen denen eine Verbindungsleitung (517) einer Schaltung mündet, die je einen Widerstand (520–521) auf jedem ihrer beiden positiven (518) und negativen (519) Zweige aufweist.

3. Gerät nach Anspruch 1, dadurch gekennzeichnet, dass die Schaltung des Senders (5) der Funksignale eine Verstärkeranordnung (508) aufweist, an der über Verbindungen (504 und 505) Leiter (502 und 503) enden, die die Kabel (3 und 4) verlängern, wobei ein einstellbarer Widerstand (533) in Reihe zu einem der Leiterelemente (502, 503, 504 oder 505) geschaltet ist, das zwischen einer Klemme (500 oder 501) und der Verstärkeranordnung (508) liegt.

4. Gerät nach Anspruch 1, dadurch gekennzeichnet, dass der Sender (5) eine Automatik aufweist, mit der die Energiequelle (6) entweder abgekoppelt ist, wenn die Elektroden (1 und 2) nicht mehr auf dem Körper des Patienten angebracht sind, oder eingeschaltet ist, wenn die Elektroden (1 und 2) sich in Betriebsstellung befinden.

5. Gerät nach Anspruch 4, dadurch gekennzeichnet, dass der Speiseausgang (534) der Energiequelle (6) an eine Schaltung (535) angeschlossen ist, die einen Zweistellungsschalter (536) aufweist, mit einem beweglichen Kontakt (537), der über Halbleiter (540 und 541) in seine festen Stellungen bewegt wird, wobei der Durchlassstrom der Halbleiter über eine Logikschaltung (542), deren Speiseeingang (543) direkt und permanent an den Ausgang (534) der Energiequelle (6) geschaltet ist, und die an eine Verbindung (505a) mit einem der Leiter (503) einer der Elektroden (2) geschaltet ist, von der Polarisation ihrer Basen abhängig gemacht ist.

0 048 187

**Fig 1**

**Fig 2**

FIG.3

FIG.4

0 048 187

FIG.6

FIG.5